Europäisches Patentamt

European Patent Office

Office européen des brevets

(11) Veröffentlichungsnummer: **0 349 745**

**A1**

## (12) EUROPÄISCHE PATENTANMELDUNG

(21) Anmeldenummer: 89109117.5

(22) Anmeldetag: 20.05.89

(51) Int. Cl.⁴: **A61M 5/14 , F16L 19/02 , F16L 47/04**

(30) Priorität: 23.06.88 DE 3821154

(43) Veröffentlichungstag der Anmeldung:
**10.01.90 Patentblatt 90/02**

(84) Benannte Vertragsstaaten:
**AT BE CH DE ES FR GB GR IT LI NL SE**

(71) Anmelder: **B. Braun Melsungen AG**
**Carl-Braun Strasse**
**D-3508 Melsungen(DE)**

(72) Erfinder: **Haindl, Hans, Dr.**
**Forstgarten 22**
**D-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Verbindungsteil mit Aussenkegel für eine verriegelbare Kegelverbindung.**

(57) Der Verbindungsteil (10) mit Außenkegel (11) für eine verriegelbare Kegelverbindung für medizinische Geräte weist einen Außenkegel (11) auf, der von einer losen Hülse (13) mit Innengewinde konzentrisch umgeben ist. Die Hülse (13) ist länger als der Außenkegel (11) und mit Rechtsgewinde (16) im vorderen Bereich versehen. Der hintere Bereich der Hülse (13) weist ein Linksgewinde (17) auf, mit dem ein äußerer Ringbund (18) des Außenkegels (11) zusammenwirkt. Auf diese Weise wird erreicht, daß sich der Ringbund des Außenkegels (11) im Anlieferungszustand am hinteren Ende des Linksgewindes (17) der Hülse (13) befindet und die Spitze des Außenkegels (11) hinter den vorderen Rand der Hülse (13) in diese zurückgezogen ist. Beim Zusammenfügen des Verbindungsteiles (10) mit Außenkegel (11) mit jedem normgerechten Verbindungsteil mit Innenkegel veranlaßt das Zusammenwirken des Ringbundes (18) mit dem Linksgewinde (17) der Hülse (13) ein Zurückschrauben der Hülse (13) relativ zum Außenkegel (11) derart, daß ihr vorderer Rand hinter die Spitze des Außenkegels (11) verlagert wird.

FIG.1

## Verbindungsteil mit Außenkegel für eine verriegelbare Kegelverbindung

Die Erfindung bezieht sich auf einen Verbindungsteil mit Außenkegel für eine verriegelbare Kegelverbindung für medizinische Geräte, bei dem der Außenkegel von einer losen Gewindemutter konzentrisch umgeben ist, die aus einer Hülse mit innerem Rechtsgewinde besteht.

Der Außenkegel eines Verbindungsteiles wird mit einem Innenkegel eines zweiten Verbindungsteiles zusammengefügt und es wird zur Verriegelung der Kegelverbindung die zylindrische Hülse der Gewindemutter auf radial nach außen abstehende gerade oder schräge Flügel am freien Ende des Innenkegels aufgeschraubt. Deartige Anschlüsse sind als Luer-Lock-Verbindungen bekannt. Das innere Rechtsgewinde der Hülse erstreckt sich bei bekannten Verbindungsteilen über die ganze axiale Länge der Hülse und DIN-Norm-gemäß steht die Spitze des Au ßenkegels mehrere Millimeter über den vorderen Rand der Hülse nach außen vor. Der Überstand des Außenkegels über die Hülse ist dadurch bedingt, daß im allgemeinen an dem Innenkegel-Verbindungsteil nicht genügend Platz für eine lange Hülse der Gewindemutter des Außenkegel-Verbindungsteils zur Verfügung steht, so daß zur Erzielung einer dichten Steckverbindung von Außenkegel und Innenkegel durch axiales Hineinziehen des Außenkegels in den Innenkegel beim Aufschrauben der Gewindemutter auf den Innenkegel-Verbindungsteil der Außenkegel länger sein muß als die Hülse der Gewindemutter. Dies trifft sowohl für fest verbundene als auch für lose an dem Außenkegel angebrachte Gewindemuttern zu. Der Überstand der Außenkegelspitze über die Hülse hat zur Folge, daß bei abgenommenem Verbindungsteile eine versehentliche Berührung des Außenkegels zur Kontamination seiner Spitze führt. Dieses Berühren tritt in der Praxis nicht selten auf und korrekterweise muß das ganze Instrument, z.B. ein Infusionsbesteck, verworfen und durch ein neues Instrument ersetzt werden.

Es ist bekannt, eine verriegelbare Kegelverbindung bei einem Verschlußstück für das rückwärtige Ende einer Leitung mit einem Außenkegel zu versehen, der kürzer ist als die fest mit ihm verbundene Hülse der Gewindemutter (DE-GM 83 03 358). Dieses Verschlußstück mit sehr kurzem Außenkegel entspricht jedoch nicht der Norm und paßt nicht in alle Innenkegel-Verbindungsteile medizinischer Geräte. Es ist nur für Spezialanwendungen mit einem Innenkegel-Verbindungsteil geeignet, dessen Innenkegel ebenfalls sehr kurz ist.

DE-OS 29 47 574 C2 offenbart einen Verbindungsteil mit einem Außenkegel, der von einer mit ihm fest verbundenen Hülse überragt wird, deren Innenwandung im Bereich ihres freien Randes keine Gewindegänge aufweist und als Dichtfläche ausgebildet ist, die mit einem Elastomerelement auf dem Außenumfang am Fuß des Innenkegels zusammenwirkt. Auch in diesem Falle handelt es sich um Spezialteile, die einander angepaßt sind und der Verbindungsteil mit Außenkegel eignet sich nicht zur Kupplung mit Norm-Verbindungsteilen mit Innenkegel.

Bei einer Doppel-Kupplung nach US-A-43 69 781 sind ein Innenkegel und ein Außenkegel nach entgegengesetzten Seiten gerichtet und mit Abstand konzentrisch von unbeweglichen Hülsen umgeben. Die Hülse des Außenkonus ist kürzer als dieser und weist ein inneres Rechtsgewinde zur Luer-Lock-Verriegelung mit radialen Flügeln am Ende eines Innenkegelteiles auf. Die Hülse des Innenkonus steht über diesen vor und trägt an ihrem freien Rand eine Scheibe mit radialen Zungen, die einen angekuppelten Außenkegelteil klemmend festhalten. Für übliche Norm-Kegelverbindungen ist diese Doppel-Kupplung auf ihrer Innenkegelseite ebenfalls nicht geeignet und auf ihrer Außenkegelseite ist der Außenkegel nicht durch die Hülse ausreichend geschützt.

Zur Erzielung eines Kontaminationsschutzes ist gemäß DE-GM 81 25 057 ein Rohrstück auf die Gewindemutter aufgesteckt worden, das länger als der über den vorderen Rand der Gewindemutter vorstehende Außenkegel ist und zur Ermöglichung des Zusammenschlusses der positiven und negativen Verbindungsteile auf der Gewindemutter nach hinten zurückziehbar angeordnet ist. Bei diesem Aufbau ist nicht zuverlässig gewährleistet, daß die Außenkegelspitze bei zurückgezogenem Rohrstück oder bei seiner Zurückziehung nicht doch kontaminierend berührt wird, bevor die Kegelverbindung hergestellt ist.

Der Erfindung liegt die Aufgabe zugrunde, einen normgerechten Verbindungsteil mit Außenkegel so auszubilden, daß die Spitze des Außenkegels gegen Kontamination durch Berührung zuverlässig geschützt ist.

Diese Aufgabe wird erfindungsgemäß dadurch gelöst, daß die Hülse länger ist als der Außenkegel und daß das Rechtsgewinde im vorderen Bereich der Hülse ausgebildet ist und der hintere Bereich der Hülse ein Linksgewinde enthält, mit dem ein äußerer Ringbund des Außenkegels zusammenwirkt.

Im Anlieferungszustand befindet sich der Ringbund des Außenkegels am hinteren Ende des Linksgewindes der Hülse und die Spitze des Außenkegels ist hinter den vorderen Rand der Hülse in diese zurückgezogen. Das vordere Ende der Hülse steht also deutlich über die Spitze des Außenkegels nach außen vor und schützt ihn gegen versehentliche Berührung und Kontamination. Beim Zusammenfügen des Verbindungsteiles mit Außenkegel mit jedem normgerechten Verbindungsteil mit Innenkegel wird das Rechtsgewinde der Hülse auf die Flügel des Innenkegel-Verbindungsteiles aufgeschraubt und dabei veranlaßt das Zusammenwirken des Ringbundes mit dem Linksgewinde der Hülse ein Zurückschrauben der Hülse relativ zum Außen kegel derart, daß ihr vorderer Rand hinter die Spitze des Außenkegels verlagert wird. Im angekoppelten Zustand beider Verbindungsteile steckt der lange Außenkegel tief und abgedichtet in dem langen Innenkegel und durch Verlegung des vorderen Randes der Hülse hinter die Außenkegelspitze ist der auf den Innenkegel-Verbindungsteil aufgeschraubte Hülsenabschnitt kurz, so daß der Verbindungsteil ohne Platz- und Abdichtungsprobleme mit allen handelsüblichen, normgerechten Verbindungsteilen mit Innenkegel koppelbar ist. Der Aufbau ist einfach und läßt sich spritztechnisch preiswürdig herstellen.

In vorteilhafter Ausgestaltung der Erfindung ist vorgesehen, daß der Ringbund auf seiner Umfangsfläche einen Linksgewindeabschnitt aufweist und daß am hinteren Ende des Linksgewindes der an beiden Enden offenen Hülse radiale Rastvorsprünge zur Halterung des Ringbundes ausgebildet sind. Der Linksgewindeabschnitt des Ringbundes hat bei der Rechtsdrehung der Hülse ihre zentrierte Führung auf dem Ringbund zur Folge, die ein verkantungsfreies Hineinziehen des Außenkegels in den Innenkegel begünstigt. Im Anlieferungszustand liegt der Ringbund zwischen zwei Rastvorsprüngen, die mit axialem Abstand angeordnet sind, der der axialen Dicke des Ringbundes etwa entspricht. Die radiale Erstreckung der Rastvorsprünge ist groß genug, um die Hülse gegen leichte axiale Verschiebung auf dem Ringbund zu sichern, jedoch gering genug, um bei Schraubverstellung der Hülse ihre Axialbewegung zu ermöglichen.

Die Hülse ist an beiden Enden offen. Dies ist erforderlich, damit der Ringbund von der rückwärtigen Seite her in das Linksgewinde bis zum Einschnappen zwischen die Rastvorsprünge einschraubbar ist. Der endseitige Rast vorsprung ist radial dicker als der innere Rastvorsprung, damit eine ungewollte Trennung der Hülse von dem Außenkegel verhindert wird und bei Betätigung der Gewindemutter eine erleichterte Überwindung des inneren Rastvorsprunges möglich ist.

In der Zeichnung ist ein Ausführungsbeispiel der Erfindung schematisch dargestellt. Es zeigen:

Fig. 1 einen Längsschnitt eines Verbindungsteiles mit Außenkegel im Anlieferungszustand und

Fig. 2 den Verbindungsteil nach Figur 1 mit angekoppeltem Innenkegel-Verbindungsteil.

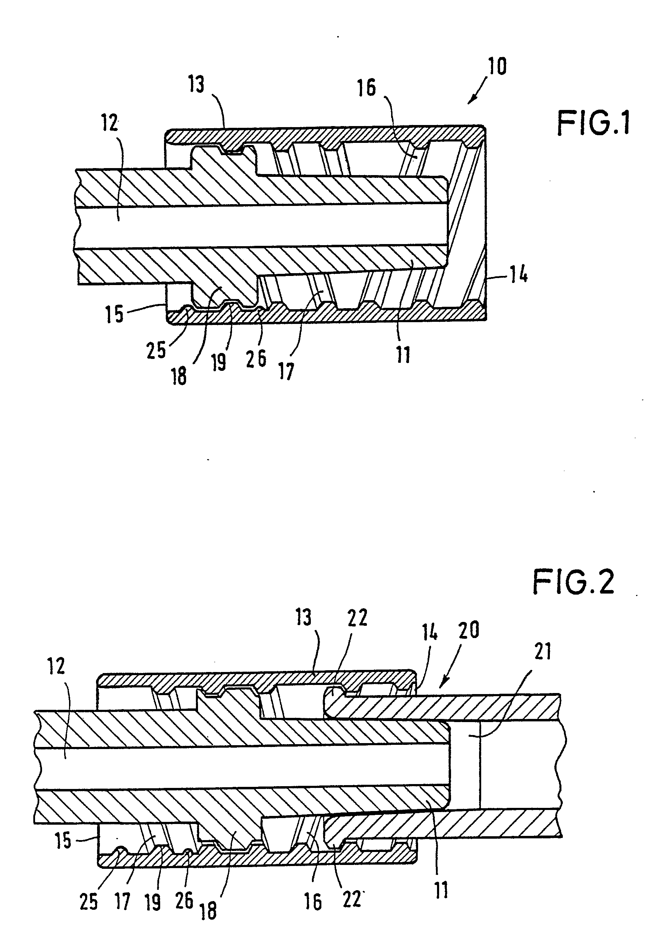

Ein Verbindungsteil 10 aus härterem Kunststoff besteht im wesentlichen aus einem Außenkegel 11 mit zylindrischem Kanal 12 und aus einer Gewindemutter in Form einer zylindrischen Hülse 13 mit Innengewinde. Die Hülse ist an ihrem vorderen Ende 14 und an ihrem hinteren Ende 15 ohne Verengung offen und das Innengewinde in ihrem zylindrischen Innenraum setzt sich aus zwei axial aufeinanderfolgenden Abschnitten zusammen, und zwar einem vorderen mehrgängigen Rechtsgewinde 16 und einem hinteren mehrgängigen Linksgewinde 17 gleicher oder ähnlicher Steigung. Das Rechtsgewinde 16 dient zum Aufschrauben der Hülse 13 auf radial nach außen gerichtete Flügel 22 eines Verbindungsteiles 20 mit Innenkegel 21, der eine Verbindung mit einem flüssigkeitsführenden Kanal, z.B. eines Infusionsbestecks, herstellt (Fig.2). Der Außenkegel 11 ist an seinem hinteren Ende von einem äußeren Ringbund 18 umgeben, dessen Außendurchmesser dem Innendurchmesser der Hülse 13 angepaßt ist und der auf seiner zylindrischen Umfangsfläche einen Linksgewindeabschnitt 19 aufweist, der mit dem Linksgewinde 17 der Hülse 13 zusammenwirkt. Der Ringbund 18 verläuft im wesentlichen in einer Ebene, die zur Längsachse des Außenkegels 11 senkrecht angeordnet ist. Im Anlieferungszustand gemäß Figur 1 ist der Ringbund 18 zwischen zwei radialen Rastvorsprüngen 25, 26 auf der Innenseite der Hülse 13 gehalten, deren axialer Abstand der axialen Dicke des Ringbundes 18 etwa entspricht. Der der hinteren Öffnung 15 näher gelegene endseitige Rastvorsprung 25 ist in radialer Richtung etwas höher als der innere Rastvorsprung 26. Es können auch mehrere Rastvorsprünge umfangsmäßig verteilt vorgesehen sein.

Zur Montage des Verbindungsteiles 10 nach Figur 1 wird auf den Linksgewindeabschnitt 19 des Ringbundes 18 linksherum die Hülse 13 aufgeschraubt, bis der Ringbund 18 zwischen den beiden Rastvorsprüngen 25, 26 im Bereich des hinteren Endes des Linksgewindes 17 einrastet. Dabei bietet der endseitige Rastvorsprung 25 einen höheren Widerstand als der flachere innere Rastvorsprung 26. In diesem Grundzustand ist die Spitze des Außenkegels 11 in die Hülse 13 zurückgezogen, d.h., ein Teil der Hülsenlänge verdeckt die Spitze des Außenkegels 11 und schützt sie gegen Kontamination durch Berührung. Das Rechtsgewinde 16 erstreckt sich von der vorderen Öffnung 14 der Hülse 13 in den Bereich der zurückgezogenen Spitze des Außenkegels 11.

Soll zur Herstellung einer Kegelverbindung der Verbindungsteil 10 mit dem Verbindungsteil 20 zusammengefügt werden, so wird die Hülse 13 mit dem Rechtsgewinde 16 durch Rechtsdrehung auf die Flügel 22 des Verbindungsteiles 20 aufgeschraubt. Die Rechtsdrehung bewirkt, daß sich dabei durch das Zusammenwirken des Linksgewindeabschnittes 19 des Ringbundes 18 des Außenkegels 11. mit dem Linksgewinde 17 der Hülse 13 diese nach hinten verstellt, so daß die Spitze des Außenkegels 11 durch die Öffnung 14 der Hülse 13 nach vorne vortritt. In gekoppeltem Zustand (Fig. 2) ergibt sich das bei den Norm-Verbindungsteilen übliche Bild des mit seiner Spitze über den vorderen Rand der Hülse vorstehenden, tief in den Innenkegel eindringenden Außenkegels und einer verhältnismäßig kurzen auf den Innenkegel-Verbindungsteil aufgeschraubten Hülsenlänge. Die recht große Länge der Hülse 13 ist nur im unbenutzten Zustand des Verbindungsteiles 10 zum Schutz des Außenkegels 11 wirksam und ist im angekoppelten Zustand soweit zurückgezogen, daß keine Behinderung des dichten Zusammenziehens von Außenkegel und Innenkegel durch axialen Platzmangel am genormten Innenkegel-Verbindungsteil auftritt.

Der Außenkegel-Verbindungsteil kann alternativ einen stopfenartig geschlossenen Außenkegel aufweisen, so daß er keine Fluidleitung herstellt, sondern das Ende einer Leitung mit Hilfe ihres Innenkegel-Verbindungsteiles dicht verschließt.

## Ansprüche

1. Verbindungsteil mit einem Außenkegel (11) für eine verriegelbare Kegelverbindung für medizinische Geräte, bei dem der Außenkegel (11) von einer losen, drehbaren Gewindemutter konzentrisch umgeben ist, die aus einer Hülse (13) mit innerem Rechtsgewinde (16) besteht, **dadurch gekennzeichnet**, daß die Hülse (13) länger ist als der Außenkegel (11), daß das Rechtsgewinde (16) im vorderen Bereich der Hülse (13) ausgebildet ist und daß der hintere Bereich der Hülse (13) ein Linksgewinde (17) aufweist, mit dem ein äußerer Ringbund (18) des Außenkegels (11) zusammenwirkt.

2. Verbindungsteil nach Anspruch 1, **dadurch gekennzeichnet**, daß der Ringbund (18) auf seiner Umfangsfläche einen Linksgewindeabschnitt (19) aufweist und daß am hinteren Ende des Linksgewindes (17) der an beiden Enden (14,15) offenen Hülse (13) radiale Rastvorsprünge (25,26) zur Halterung des Ringbundes (18) ausgebildet sind.

3. Verbindungsteil nach Anspruch 2, **dadurch gekennzeichnet**, daß zwei Rastvorsprünge (25,26) mit einem axialen Abstand angeordnet sind, der der axialen Dicke des Ringbundes (18) etwa entspricht, und daß der endseitige Rastvorsprung (25) radial dicker als der innere Rastvorsprung (26) ist.

FIG.1

FIG.2

Europäisches
Patentamt

# EUROPÄISCHER RECHERCHENBERICHT

Nummer der Anmeldung

EP 89 10 9117

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | US-A-4 369 781 (GILSON et al.)<br>--- | | A 61 M 5/14<br>F 16 L 19/02<br>F 16 L 47/04 |
| A,D | GB-A-2 040 379 (BAXTER TRAVENOL)<br>& DE-A-2 947 574<br>--- | | |
| A | GB-A-2 089 921 (BAXTER TRAVENOL)<br>--- | | |
| A | US-A-4 046 479 (PALEY)<br>----- | | |

RECHERCHIERTE
SACHGEBIETE (Int. Cl.4)

A 61 M
F 16 L
H 01 R

Der vorliegende Recherchenbericht wurde für alle Patentansprüche erstellt

| Recherchenort | Abschlußdatum der Recherche | Prüfer |
|---|---|---|
| DEN HAAG | 03-10-1989 | CLARKSON P.M. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
.............................................................
& : Mitglied der gleichen Patentfamilie, übereinstimmendes
   Dokument